# EUROPÄISCHE PATENTSCHRIFT

(11) **EP 1 014 943 B1**
(45) Veröffentlichungstag und Bekanntmachung des Hinweises auf die Patenterteilung: **19.06.2002**
(21) Anmeldenummer: 98900837.0
(22) Anmeldetag: 02.02.1998
(51) Int. Cl.: A61K 9/12, A61K 9/00

(54) **MEDIZINISCHE AEROSOLFORMULIERUNGEN**
MEDICAL AEROSOL FORMULATIONS
FORMULATIONS MEDICALES POUR AEROSOLS

(30) Priorität: 05.02.1997 CH 24897
(43) Veröffentlichungstag der Anmeldung: 05.07.2000
(73) Patentinhaber: JAGO RESEARCH AG, CH-4132 Muttenz (CH)
(72) Erfinder: KELLER, Manfred, D-79189 Bad Krozingen (DE); HERZOG, Kurt, CH-4055 Basel (CH)
(74) Vertreter: Zimmermann, Hans, Dr.
(86) Internationale Anmeldenummer: CH9800037
(87) Internationale Veröffentlichungsnummer: WO9834595

(56) Entgegenhaltungen:
- WO-A-93/17665
- WO-A-94/01511
- US-A- 4 139 607
- DATABASE WPI Week 8635 Derwent Publications Ltd., London, GB; AN 86-228980 [35] XP002039614 in der Anmeldung erwähnt & JP 61 158 919 A (TAISHO PHARM. CO. LTD. ) 18.Juli 1986
- DATABASE WPI Week 8925 Derwent Publications Ltd., London, GB; AN 89-184245 [25] XP002039615 in der Anmeldung erwähnt & ANONYMOUS: "AEROSOL PRESSURE PACKS FOR ADMINISTRATION OF MEDICAMENTS CONTAIN PROPELLANTS SUCH AS HYDROCARBON(S),CARBON DIOXIDE,NITROGEN ETC. AND/OR FLUOROHYDROCARBON(S)" RESEARCH DISCLOSURE, Nr. 301061, 10.Mai 1989,

## Beschreibung

Die vorliegende Erfindung betrifft eine medizinische Aerosolformulierung, die ein druckverflüssigtes Treibmittelgemisch auf der Basis von Hydrofluoralkanen enthält, sowie ein Verfahren zur Herstellung der Aerosolformulierung.

Viele Gase, wie z.B. Kohlendioxid und Stickstoff, lassen sich zwar unter Druck verflüssigen, eignen sich aber nicht als Treibmittel für Dosieraerosole, weil der Binnendruck im Behältnis mit zunehmender Entleerung sehr stark abnimmt. Aus diesem Grunde eignen sich nur solche Treibgase für medizinische Dosieraerosole, die sich bei Raumtemperatur verflüssigen lassen und nur zu einer geringfügigen Abnahme des Binnendrucks führen, wenn der Inhalt sukzessive abgesprüht wird. Hierzu zählen die treibgasförmigen Alkane, wie z.B. Propan, Butan und Isobutan, sowie die Fluorchlorkohlenwasserstoffe (FCKWs), wie z.B. Trichlorfluormethan (F11), Dichlordifluormethan (F12) und 1,2-Dichlor-1,1,2,2-tetrafluorethan (F114).

Für Aerosolanwendungen wie Haarsprays, Deodorantsprays und dergleichen wurden gelegentlich auch Kombinationen von Treibgasen vorgeschlagen. Beispielsweise offenbart WO-A-94/01511 Aerosolformulierungen aus einem komprimierten Gas (Stickstoff, Kohlendioxid, Druckluft, Sauerstoff, Xenon und/oder Argon), einem verflüssigten Kohlenwasserstofftreibmittel, Wirkstoff und Träger, wobei die Formulierungen typischerweise 0,05-2,5 Gew.-% Stickstoff und 1,0-12,0 Gew.-% verflüssigtes Kohlenwasserstofftreibmittel enthalten können und vorzugsweise einen Gehalt von 80-95 Gew.-% an flüchtigen Trägerverbindungen, wie Ethanol, Propanol, Pentan, Wasser, Aceton und dergleichen, aufweisen. Im Derwent-Abstract AN 86-228980 wird ferner ein Dermatophytiemittel beschrieben, welches 0,1-2 Gew.-% Tolunaphtat, 0,5-70 Gew.-% Treibmittel und 30-80 Gew.-% fluoriertes Alkylhalogenid (Trichlormonofluormethan, Tetrachlordifluorethan, Trichlortrifluorethan und/oder Dibromtetrafluorethan) mit einem Siedepunkt von mindestens 20°C als Lösungsmittel enthält; als Treibmittel sollen sich Petroleumgas, Dimethylether, Dichlordifluormethan, Dichlortetrafluorethan, Kohlendioxid etc. eignen. Andererseits ist aus WO-A-93/17665 eine Methode zur Verabreichung physiologisch wirksamer Verbindungen bekannt, bei der aus einem überkritischen flüssigen Lösungsmittel und dem Wirkstoff eine überkritische flüssige Lösung gebildet und diese dann in den unterkritischen Bereich übergeführt wird. Als überkritische Lösungmittel sollen sich Kohlendioxid, Distickstoffmonoxid, Fluorchlorkohlenwasserstoffe, Xenon, Schwefelhexafluorid, Ethanol, Aceton, Propan und/oder Wasser eignen.

Aufgrund der Ozonproblematik, hervorgerufen durch die Abspaltung von radikalischen Chloratomen aus den FCKWs, haben sich im Montrealer Abkommen viele Staaten darauf verständigt, die FCKWs als Treibmittel zukünftig nicht mehr zu verwenden. Als FCKW-Ersatzstoffe für den medizinischen Bereich eignen sich fluorierte Alkane, vor allem 1,1,1,2-Tetrafluorethan (HFA 134a) und 1,1,1,2,3,3,3-Heptafluorpropan (HFA 227), da diese inert sind und eine sehr geringe Toxizität aufweisen. Aufgrund ihrer physikalischen Eigenschaften, wie Druck, Dichte etc. sind sie besonders geeignet, um die FCKWs wie F11, F12 und F114 als Treibmittel in Dosieraerosolen zu ersetzen.

In US-A-4 139 607 wurde ferner ein Treibmittelsystem aus verflüssigtem Bis(difluormethyl)ether und gasförmigem Kohlendixoid vorgeschlagen, das im Gegensatz zu Kombinationen von Kohlendioxid mit anderen bekannten Treibmitteln wie Trichlorfluormethan oder Methylenchlorid befriedigende Aerosolmuster ergeben soll, das sich aber nicht durchgesetzt hat. Gemäss der Offenbarung von US-A-4 139 607 können dem Treibmittelsystem andere, herkömmliche Treibmittel wie Distickstoffmonoxid, Kohlenwasserstoffe und Fluorkohlenwasserstoffe oder flüssige Träger, wie Ethanol, Perchlorethylen, Trichlorethylen, Aceton, Amylacetat, Wasser und dergleichen, zugesetzt werden, wobei in den offenbarten Beispielen meist Ethanol und Bis(difluormethyl)ether im Gewichtsverhältnis von etwa 1:1 verwendet wurden. Andererseits wird im Derwent-Abstract AN 89-184245 ausgesagt, dass in Aerosoldruckpackungen zur Verabreichung von Medikamenten anstelle von FCKWs auch Kohlenwasserstoffe, wie Butane und Pentane, andere Druckgase, wie Kohlendioxid, Dimethylether, Stickstoff und Distickstoffoxid, oder Fluorkohlenwasserstoffe verwendet werden könnten.

FCKW-freie medizinische Aerosolzubereitungen mit HFA 134a sind bereits durch die allgemeine Lehre von US-A-2 868 691 und US-A-3 014 844 mit umfasst und aus DE-A-2 736 500 und EP-A-0 372 777 bekannt. Beispiele mit HFA 227 finden sich in WO-A-91/11495, EP-A-0 504 112 und EP-B-0 550 031. Es ist aus verschiedenen Veröffentlichungen bekannt, dass sich die üblichen, in FCKW-haltigen Dosieraerosolen verwendeten Hilfsstoffe, wie z.B. Lecithin, Sorbitantrioleat und Ölsäure, nur unzureichend in Hydrofluoralkanen (im Rahmen der vorliegenden Erfindung mit "HFA" bezeichnet), wie z.B. HFA 134a und HFA 227 lösen, weil eine Kettenverlängerung und die Substitution der Chloratome durch Fluoratome zu einer Verschlechterung der Löslichkeitseigenschaften für polare Stoffe führt. Bereits bei den FCKWs, die im Vergleich zu den HFAs erheblich bessere Lösungsmittel darstellen, wurden zur Verbesserung der Löslichkeit oft Ethanol oder andere Cosolventien zugesetzt, um Arzneistoffe wie z.B. Isoprenalin und Epinephrine (vgl. US-A-2 868 691) als Aerosol applizieren zu können. Es war daher naheliegend, nicht nur die Löslichkeit der FCKWs, sondern auch diejenige der HFAs durch Zugabe von Ethanol zu verbessern. Beispiele hierfür finden sich in der Fachliteratur als auch in verschiedenen Patentanmeldungen. Alternativ dazu gibt es eine .Reihe von Entwicklungen druckverflüssigter Aerosolzubereitungen mit HFA 134a und/oder HFA 227, die treibgaslösliche Hilfsstoffe, wie z.B. fluorierte oberflächenaktive Stoffe (WO-A-91/04011), mono- oder diacetylierte Glyceride (EP-A-0 504 112) oder polyethoxylierte Verbindungen (WO-A-92/00 061), verwenden, die sich auch ohne Ethanolzusatz in den beiden Treibgasen in der erforderlichen Menge lösen lassen. Bisher ist jedoch erst ein Produkt auf der Basis von HFAs als bioäquivalenter Ersatz zugelassen worden, nämlich eine Suspensionsaerosolformulierung von Salbutamolsulfat in HFA 134a, Ethanol und Ölsäure (Airomir®, 3M Health Care Ltd., England).

Für Neuentwicklungen medizinischer, FCKW-freier Aerosolzubereitungen werden heute als Treibgase vorzugsweise Hydrofluoralkane wie HFA 134a (Dampfdruck ca. 6 bar bei 20°C) und HFA 227 (Dampfdruck ca. 4,2 bar bei 20°C) verwendet. Beide Treibgase unterscheiden sich hinsichtlich ihrer Dichte (ca. 1,4 mg/ml für HFA 227 und 1,2 mg/ml für HFA 134a bei 20°C), was insbesondere für Suspensionen von Bedeutung ist. Besitzt der Wirkstoff eine höhere Dichte als das Treibgas, kommt es zu einer Sedimentation, ist dessen Dichte geringer, kommt es zu einer Flotation. Zur Problemlösung bietet es sich deshalb unter Umständen an, Treibgas-Mischungen zu verwenden und/oder zur Erniedrigung der Dichte Cosolventien wie Ethanol, Diethylether oder andere niedrig siedende Lösungsmittel oder Treibgase wie n-Butan zuzusetzen. Ein wesentlicher Nachteil der HFAs ist deren geringeres Lösungsvermögen im Vergleich zu den FCKWs, insbesondere im Vergleich zu F11. Die Solvenzeigenschaften nehmen mit zunehmender Kettenlänge in der Reihenfolge F11 > HFA 134a > HFA 227 ab. Aus diesem Grunde lassen sich ohne Erhöhung der Hydrophilie durch Zugabe von polaren Lösungsmitteln, wie z.B. Ethanol, die üblicherweise in FCKWs verwendeten Suspendierhilfsmittel, wie Sorbitantrioleat, Lecithin und Ölsäure, nicht mehr in den üblichen Konzentrationen (ca. 1 : 2 bis 1 : 20, bezogen auf den Wirkstoff) lösen und damit verwenden.

Es ist allgemein bekannt, dass im Falle von Suspensionsformulierungen nur Wirkstoffteilchen, die kleiner als 6 µm sind, lungengängig sind. Zur gewünschten Deposition derselben in der Lunge, müssen diese deshalb vor der Verarbeitung mittels spezieller Verfahren, wie z.B. Mikronisierung (Mahlen), zerkleinert werden. Der Mahlprozess führt bei den meisten Wirkstoffen zu einer Oberflächenvergrösserung, die meist mit einer Erhöhung der elektrostatischen Ladung einhergeht. Dies kann in der Aerosolzubereitung zu einer Agglomeration oder Koagulation führen und erschwert im allgemeinen eine homogene Wirkstoff-Dispergierung. Als Folge der Grenz- und Ladungsaktivitäten kommt es häufig zu einer Adsorption von Wirkstoff an Grenzflächen, die z.B. zu einer Ringbildung im Behälter an der Stelle führt, wo die Flüssigphase in die Gasphase übergeht. Daneben lassen sich die Suspensionen oft nur unzureichend stabilisieren bzw. in dispergiertem Zustand halten, was ebenfalls mit einer Veränderung der Dosis pro Hub verknüpft sein kann, wenn vor Gebrauch, d.h. vor der Inhalation, die Suspension nicht durch Schütteln homogen redispergiert werden kann. Die mangelhafte Benetzung bzw. Dispergierung der Wirkstoffpartikel hat auch zur Folge, dass diese in vielen Fällen eine hohe Adsorptionstendenz aufweisen und an Oberflächen, wie z.B. der Behälterinnenwand oder dem Ventil, kleben, was dann zu einer vermeintlichen Unterdosierung sowie schlechten Dosiergenauigkeit im Sprühstoss bzw. der "Content Uniformity" (CU, Gleichförmigkeit des Gehalts) der deklarierten Anzahl von z.B. 200 oder 300 Sprühstössen (d.h. Einzeldosen) führt. Bei Suspensionen ist es deshalb in der Regel erforderlich, einen oberflächenaktiven Hilfsstoff bzw. ein Schmiermittel zuzusetzen, weil es ansonsten zu einem Klemmen der Ventile und einer ungenauen Abgabe des Wirkstoffs von Sprühstoss zu Spühstoss kommen kann. Besonders problematisch ist eine im Laufe der Lagerung eintretende Veränderung bzw. Erniedrigung der inhalierbaren, lungengängigen Teilchen, der sogenannten "Fine Particle Fraction" (FPF), was zu einer Abnahme der Wirksamkeit der HFA-Zubereitung führt.

Zur Überwindung der oben dargelegten Probleme können z.B. durch pharmazeutisch-technologische Massnahmen die Benetzungs- und Löslichkeitseigenschaften des Wirkstoffes verändert In der Regel werden jedoch oberflächenaktive Substanzen zugesetzt, wie sie bereits früher bei den FCKW-haltigen Formulierungen Anwendung fanden. Weil sich aber oberflächenaktive Mittel wie Ölsäure und Lecithin nur unzureichend in Hydrofluoralkanen wie HFA 134a und/oder HFA 227 lösen, wird meistens ein Cosolvens wie Ethanol zugesetzt, damit man die pharmazeutisch-technologischen Probleme besser kontrollieren kann.

Werden höhere Ethanolkonzentrationen zugesetzt, erniedrigt sich die Dichte der Treibgas-Mischung, was vor allem bei Suspensionen zu einer unerwünschten Wirkstoff-Sedimentation führen kann. Daneben kann es aber durch die Erhöhung der Löslichkeit während der Lagerung auch zu Anlösungseffekten kommen, was dann zu einem Kristallwachstum führt. Dieser Effekt als auch eine Agglomeration der Wirkstoffpartikel kann dann im Laufe der Lagerung zu einer Veränderung bzw. Erniedrigung der inhalierbaren, lungengängigen Teilchen, der sogenannten "Fine Particle Fraction" (FPF), führen.

Zur Messung der aerodynamischen Partikelgrössenverteilung und des Anteils, der Masse oder der Dosis der inhalierbaren, lungengängigen Teilchen (d.h. der Fine Particle Fraction FPF, Fine Particle Mass FPM bzw. Fine Particle Dose FPD) eignen sich Impaktoren, wie z.B. der 5-Stufen Multistage Liquid Impinger (MSLI) oder der 8-Stufen Andersen Kaskaden Impaktor (ACI), die in Chapter <601> der United States Pharmacopoeia (USP) oder in der Inhalanda Monographie der Europäischen Pharmacopoe (Ph. Eur.) beschrieben sind. Anhand der aerodynamischen Partikelgrössenverteilung kann man mittels eines "Log-probability plots" (logarithmische Darstellung der Wahrscheinlichkeitsverteilung) den mittleren aerodynamischen Teilchendurchmesser (Mass Median Aerodynamic Diameter MMAD) von Aerosol-Zubereitungen abschätzen und ableiten, ob der Wirkstoff eher im oberen oder unteren Lungenbereich deponiert wird.

Liegt der Wirkstoff im HFA-Treibgas/Ethanol-Gemisch nicht suspendiert, sondern gelöst vor, sind Probleme in Bezug auf die Streuung der Dosiergenauigkeit pro Hub meist weniger ausgeprägt.

Bei gleichem Ethanolgehalt erhält man mit HFA 134a im Vergleich zu HFA 227 einen grösseren Prozentsatz inhalierbarer (kleinerer) Teilchen, was auf den höheren Druck von HFA 134a zurückzuführen ist. Prinzipiell gilt: Je höher der Binnendruck in der Aerosoldose, desto feiner ist das Teilchenspektrum der Aerosolwolke. Lösungsaerosole mit geringem Ethanolanteil weisen in der Regel bei Verwendung von feinen Vernebelungsdüsen auch einen kleineren MMAD (0,8 -1,5 µm) auf als Suspensionsaerosole (2 - 6 µm). Dies hängt damit zusammen, dass bei Lösungsaerosolen Tröpfchen und bei Suspensionsaerosolen Partikel erzeugt werden.

Für die topische Applikation von Wirkstoffen im Bereich der Bronchien und Bronchiolen, sind Partikelgrössen von ca. 2 - 4 µm vorteilhaft, wie sie üblicherweise mit Suspensionsformulierungen erreicht werden. Kleinere Partikel, die in den Alveolarbereich gelangen, werden zum Teil exhaliert (< 0,5 µm) oder gelangen durch Absorption in den systemischen Kreislauf. Hieraus folgt, dass Aerosolzubereitungen für die systemische Applikation günstigerweise Partikelgrössen von ca. 0,5 µm - 2 µm aufweisen sollten, wobei z.B. ein monodisperses Aerosol mit einem sehr hohen Anteil an Partikeln im Bereich von ca. 1 µm besonders vorteilhaft wäre. Abhängig vom gewünschten Depositionsort ist deshalb ein kleinerer oder grösserer MMAD sowie gegebenenfalls ein monodisperses Verteilungsspektrum bevorzugt. Hinsichtlich der Aerodynamik gilt: Je grösser die Masse der Partikel desto grösser ist ihre Tendenz geradlinig weiterzufliegen. Hieraus ergibt sich, dass es bei einer Änderung der Strömungsrichtung zur Impaktion von Teilchen kommt. Aus Depositionsstudien ist bekannt, dass selbst bei einem optimalen Inhalationsmanöver nur ca. 20% der aus einem Dosieraerosol emittierten Teilchen in die Lunge gelangen und nahezu 80 % im Oropharynx impaktieren.

Bei ethanolhaltigen Lösungsaerosolen kommt es leider häufig zu Problemen betreffend der Wirkstoffstabilität. Der Zersetzungsgrad von Wirkstoffen, wie z.B. Budesonid, Fenoterol-HBr, Formoterol-fumarat, Ipratropiumbromid, Salbutamol etc., kann nach Lagerung in ethanolhaltigen Lösungsaerosolen über den tolerierten Höchstwerten (< 0,5%) liegen. Nachteilig ist des weitern, dass bei höheren Ethanolkonzentrationen von z.B. 10% - 30% der Anteil inhalierbarer Partikel (< 6 µm) abnimmt, weil aufgrund der andersgearteten Verdampfungseigenschaften von Ethanol weniger Energie zur Dispergierung der Aerosolzubereitung, d.h. zur Bildung inhalierbarer Tröpfchen bzw. Partikel bereitgestellt wird. Diese und andere Gründe geben einen Hinweis darauf, weshalb die meisten Dosieraerosole als Suspensionen in den Handel gebracht wurden.

Aufgrund der oben dargelegten Zusammenhänge wäre es also wünschenswert für Dosieraerosole, ein Treibgassystem zu haben, mit dem man:
- Wirkstoffe besser benetzen kann;
- Suspensionsaerosole mit verbesserten Suspensions- und Haltbarkeitseigenschaften herstellen kann;
- Lösungsaerosole mit verbesserter Lagerungsstabilität und geringerem Ethanolzusatz herstellen kann;
- die Dosiergenauigkeit verbessern kann;
- die Partikelgrösse bzw. das Partikelgrössenverteilungsspektrum besser einstellen kann; und
- die Fine Particle Dose erhöhen kann.

Diese Aufgabe wird erfindungsgemäss gelöst durch Verwendung eines Treibgasgemisches auf der Basis von Kohlendioxid und mindestens eines Hydrofluoralkans mit 1 bis 3 Kohlenstoffatomen.

Es wurde nämlich gefunden, dass sich Kohlendioxid in den Hydrofluoralkanen löst und quasi seine Eigenschaften als kompressibles Gas verliert. Derartige Hydrofluoralkan/Kohlendioxid-Treibgasgemische zeigen bei zunehmender Entleerung nur eine geringfügige Abnahme des Binnendrucks im Behältnis, was deren Verwendung als Treibmittel für Dosieraerosole ermöglicht. Durch die Zugabe von Kohlendioxid zu den Hydrofluoralkanen erhöht sich der Druck annähernd linear mit steigender Kohlendioxidkonzentration. Ferner wird der Druckverlauf in Abhängigkeit der Temperatur durch den Gehalt an Kohlendioxid dahingehend beeinflusst, dass es im Gegensatz zu reinen Hydrofluoralkanen (vgl. Fig. 2 von EP-B-0 550 031) nur zu einem schwach exponentiellen, nahezu linearen Druckanstieg kommt, wenn die Temperatur schrittweise z.B. von 20°C auf 50°C erhöht wird. Diese Effekte werden auch beobachtet, wenn das Treibgasgemisch zusätzlich ein Cosolvens enthält.

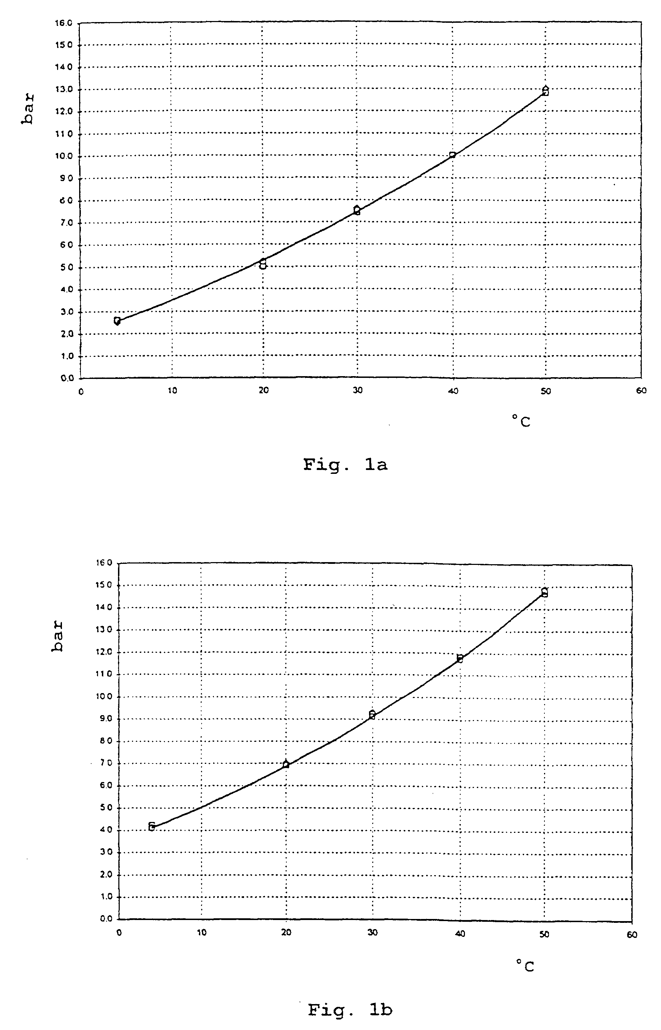

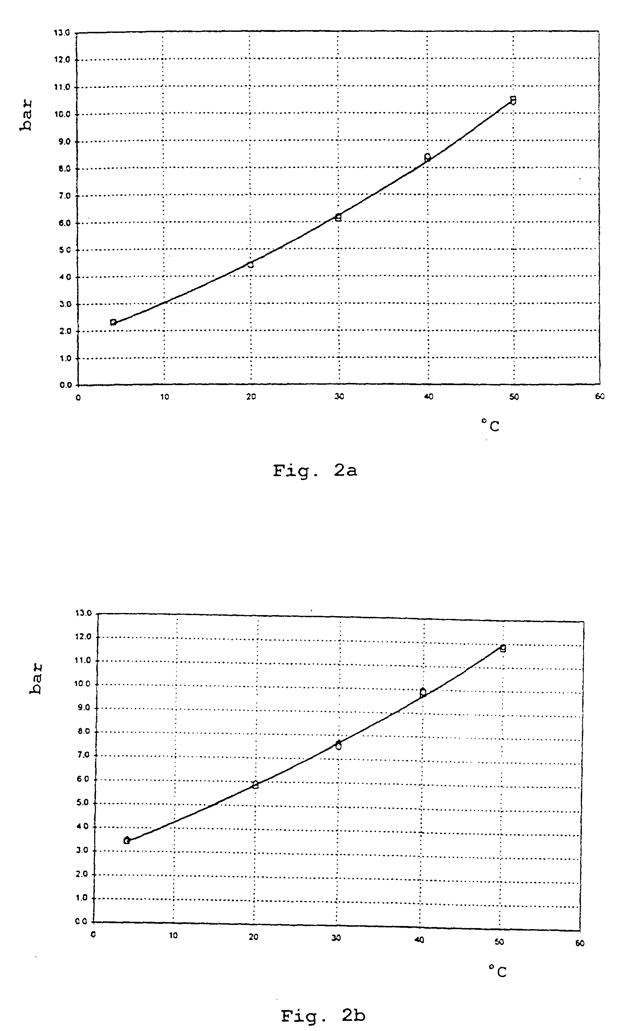

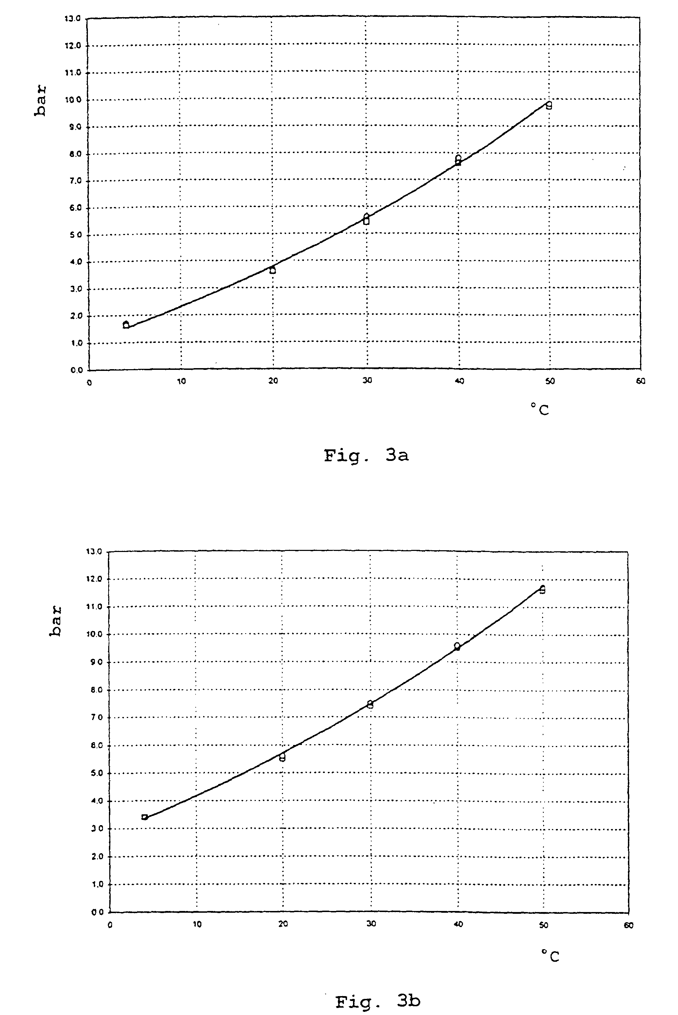

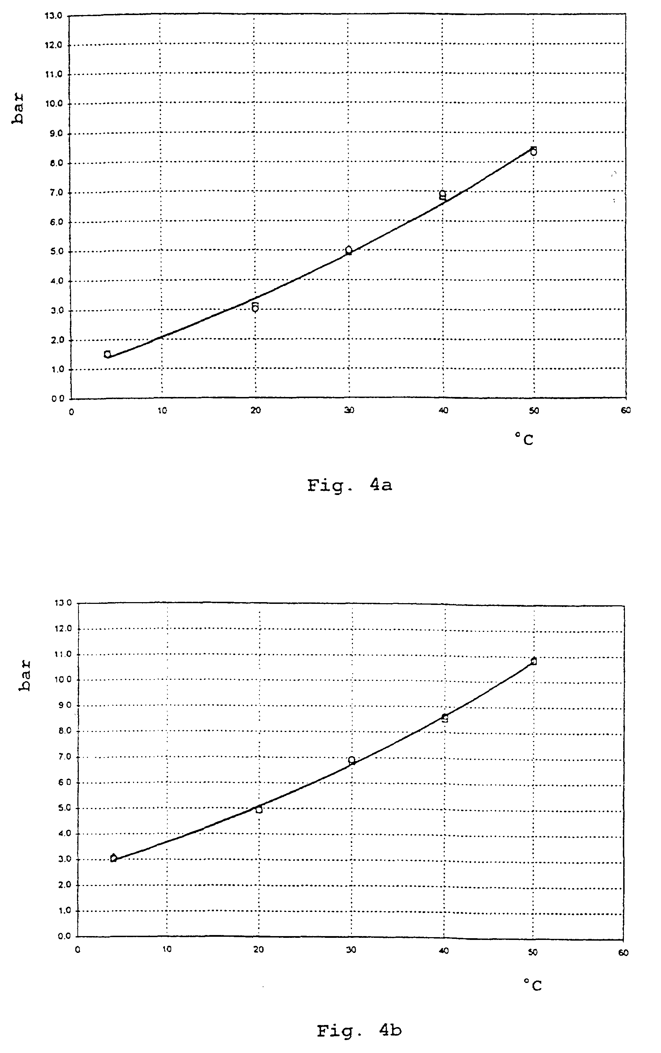

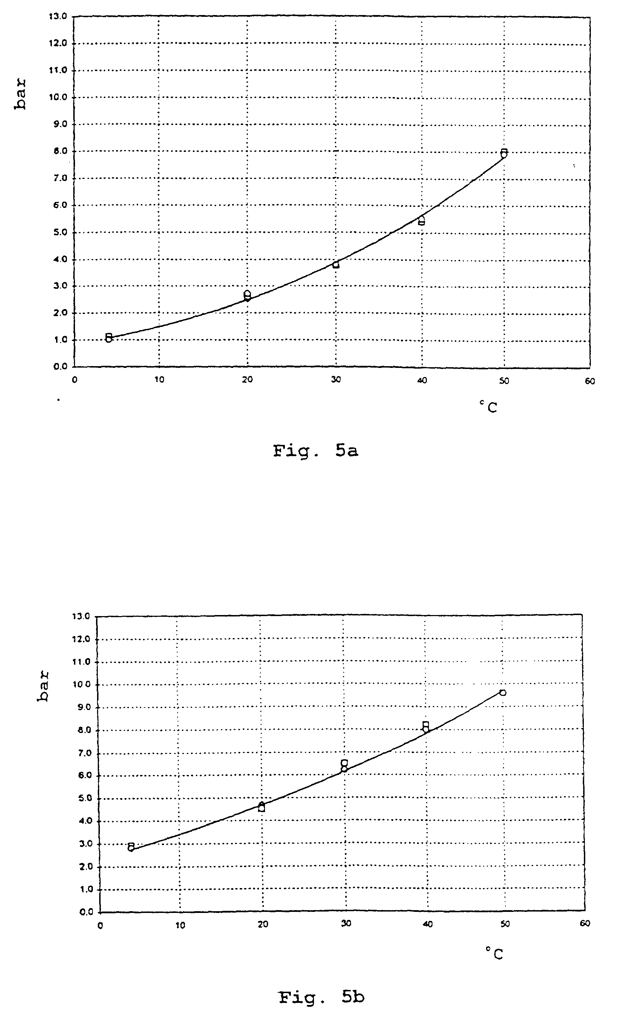

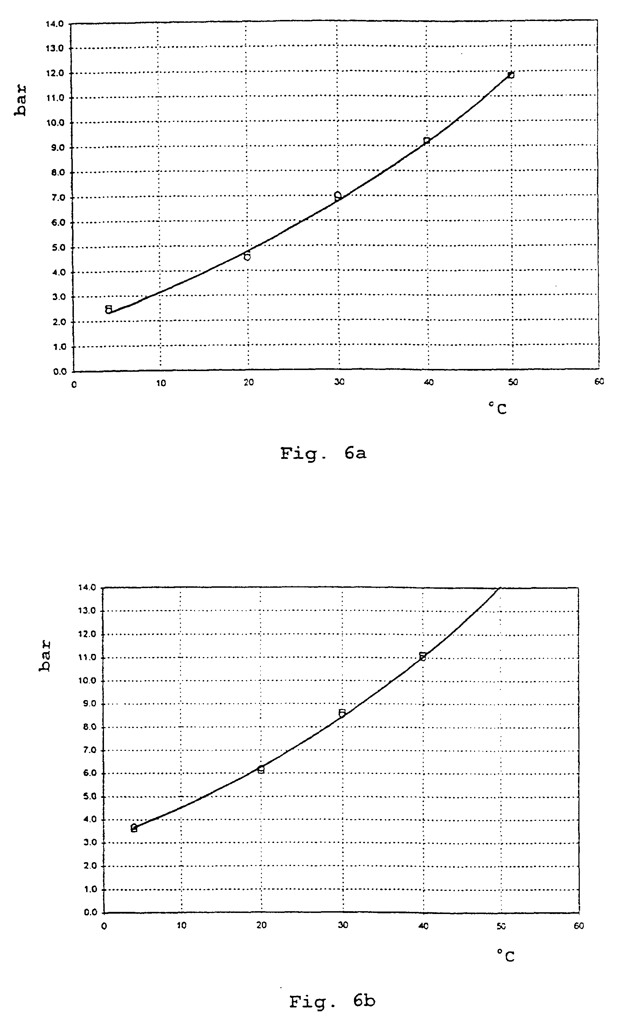

Fig. 1-6 zeigen graphische Darstellungen des Druckverlaufes in Abhängigkeit von der Temperatur für Beispiele erfindungsgemässer Treibmittelgemische, nämlich
- Fig. 1a und 1b für ein Treibmittelgemisch aus 300 Gewichtsteilen HFA 134a und 1 bzw. 3 Gewichtsteilen Kohlendioxid,
- Fig. 2a und 2b für ein Treibmittelgemisch aus 210 Gewichtsteilen HFA 134a, 90 Gewichtsteilen Ethanol und 1 bzw. 3 Gewichtsteilen Kohlendioxid,
- Fig. 3a und 3b für ein Treibmittelgemisch aus 300 Gewichtsteilen HFA 227 und 1 bzw. 3 Gewichtsteilen Kohlendioxid,
- Fig. 4a und 4b für ein Treibmittelgemisch aus 277,5 Gewichtsteilen HFA 227, 22,5 Gewichtsteilen Ethanol und 1 bzw. 3 Gewichtsteilen Kohlendioxid,
- Fig. 5a und 5b für ein Treibmittelgemisch aus 210 Gewichtsteilen HFA 227, 90 Gewichtsteilen Ethanol und 1 bzw. 3 Gewichtsteilen Kohlendioxid, und
- Fig. 6a und 6b für ein Treibmittelgemisch aus 180 Gewichtsteilen HFA 134a, 120 Gewichtsteilen HFA 227 und 1 bzw. 3 Gewichtsteilen Kohlendioxid.

Da sich der Treibgasdruck CO₂-haltiger Hydrofluoralkane nicht im Quadrat, sondern nur annähernd linear erhöht, sind deshalb derartige Treibgassysteme weitaus weniger temperaturabhängig als zum Beispiel reine FCKW-Gemische oder Hydrofluoralkane wie HFA 134a und/oder HFA 227.

Überraschenderweise wurde zusätzlich gefunden, dass die Zugabe von Kohlendioxid zu Hydrofluoralkanen wie HFA 134a und/oder HFA 227 zu einer wesentlichen Verbesserung der Benetzungseigenschaften für pharmazeutische Wirkstoffe führt. Durch Einleiten von Kohlendioxid werden also ähnlich einem Wirkstoffcoating Hydrofluoralkane dahingehend in ihren Benetzungseigenschaften verändert, dass man die mit HFA 134a, HFA 227 und anderen Hydrofluoralkanen bestehenden Formulierungsprobleme in Bezug auf Suspensions- als auch Lösungsaerosole überwinden kann. Schon eine Zugabe von Kohlendioxid in einer Grössenordnung von 0,2 Gew.-% oder weniger reduziert signifikant die Adhäsionstendenz von Wirkstoffen an Grenzflächen und vermindert deren Adsorption, Agglomeration und Koagulation. Bereits durch Zugabe geringer Mengen von Kohlendioxid werden die Hydrofluoralkane gewissermassen hydrophilisiert. Damit lassen sich von vielen Wirkstoffen ohne weitere Hilfsmittel Suspensionen herstellen, die sich durch kontrollierte Flockung auszeichnen. Als Folge der besseren Suspendiereigenschaften kann man in vielen Fällen auf die Zugabe von zum Teil unerwünschten oberflächenaktiven Suspendierhilfsmitteln wie z.B. Ölsäure oder eines Cosolvens wie z.B. Ethanol verzichten.

Das erfindungsgemäss verwendbare Treibmittelgemisch bietet jedoch auch bei Aerosolformulierungen Vorteile, bei denen ein oberflächenaktives Mittel und/oder ein Cosolvens nötig oder erwünscht ist. Einerseits gestattet die Verwendung der kohlendioxid-haltigen Treibmittel nämlich häufig eine Verringerung der benötigten Cosolvensmenge und eine bessere Löslichkeit herkömmlicher oberflächenaktiver Mittel. Anderseits kann der nachteilige Einfluss von Cosolventien wie Ethanol auf die Tröpfchengrösse ganz oder weitestgehend vermieden werden, da durch entsprechende Erhöhung der Kohlendioxidkonzentration auch bei vergleichsweise hohen Cosolvenskonzentrationen der Binnendruck so eingestellt werden kann, dass eine ausreichende Fine Particle Dose erreicht werden kann.

Mit Hilfe von Kohlendioxid ist es auch möglich, Sauerstoff aus den Hydrofluoralkanen zu verdrängen, wodurch die Lagerstabilität von oxidationsempfindlichen Wirkstoffen verbessert wird. Darüberhinaus kann man mit Kohlendioxid den Binnendruck im Aerosolbehältnis so einstellen, dass man im Vergleich zu einem konventionellen FCKW- oder HFA-Dosieraerosol, die FPF und den MMAD quasi so beeinflussen kann, wie es für die Anwendung am sinnvollsten erscheint. Es ist somit möglich, sowohl MDIs (Metered Dose Inhalers) für die lokale als auch topische Applikation herzustellen. Insbesondere für die systemische Applikation eröffnen sich völlig neue Anwendungsmöglichkeiten, weil man in Verbindung mit geeigneten Vernebelungsdüsen quasi monodisperse Aerosole mit definiertem MMAD und hohen respirablen Fraktionen herstellen kann.

Ein druckverflüssigtes Treibmittelgemisch, umfassend Kohlendioxid und ein Hydrofluoralkan der allgemeinen Formel

CₓH_{y}F_{z} (I)

worin x für die Zahl 1, 2 oder 3 steht, y und z je
eine ganze Zahl ≥ 1 bedeuten und y + z = 2x + 2 ist, eignet sich daher als Treibgas für Aerosole.

Die Herstellung der Treibmittelgemische kann in an sich bekannter Weise dadurch erfolgen, dass man Kohlendioxid unter Druck in ein Hydrofluoralkan der Formel I einleitet.

Das Treibmittelgemisch eignet sich grundsätzlich für beliebige Aerosolanwendungen wie beispielsweise kosmetische und Haushaltssprays. Aufgrund der beschriebenen Vorteile - wie geringer Abfall des Binnendruckes bei Entleerung, geringere Temperaturabhängigkeit und leichtere Einstellbarkeit des Binnendruckes, verbesserte Benetzungseigenschaften für pharmazeutische Wirkstoffe und Verwendbarkeit herkömmlicher oberflächenaktiver Mittel wie Ölsäure, Lecithin und Sorbitantrioleat - ist das Treibmittelgemisch aber vor allem auch für medizinische Aerosolformulierungen und insbesondere für Inhalationsaerosole geeignet.

Die Erfindung betrifft daher eine medizinische Aerosolformulierung, umfassend eine wirksame Menge eines pharmazeutischen Wirkstoffes und ein druckverflüssigtes Treibmittelgemisch wie oben definiert.

Beispiele geeigneter Hydrofluoralkane, die in den erfindungsgemässen Treibmittelgemischen und Aersolformulierungen verwendet werden können, sind: Difluormethan (HFA 32), Pentafluorethan (HFA 125), 1,1,2,2-Tetrafluorethan (HFA 134), 1,1,1,2-Tetrafluorethan (HFA 134a), 1,1,2-Trifluorethan (HFA 143), 1,1,1-Trifluorethan (HFA 143a), 1,1-Difluorethan (HFA 152a), 1,1,1,2,3,3,3-Heptafluorpropan (HFA 227), Hexafluorpropan (HFA 236), Pentafluorpropan (HFA 245) und dergleichen. Im allgemeinen sind Hydrofluoralkane mit 2 oder 3 Kohlenstoffatome bevorzugt. Besonders bevorzugt sind Treibmittelgemische und Aerosolformulierungen, die 1,1,1,2-Tetrafluorethan (HFA 134a), 1,1,1,2,3,3,3-Heptafluorpropan (HFA 227) oder ein Gemische der beiden, beispielsweise ein 1:1-Gemisch, enthalten.

Der Kohlendioxidgehalt der Treibmittelgemische und Aerosolformulierungen kann vorzugsweise etwa 0,0001 bis 10 Gew.-% betragen, wobei in der Regel Konzentrationen von bis zu etwa 6 Gew.-%, insbesondere bis zu etwa 3 Gew.-% besonders bevorzugt sind. In den meisten Fällen ist eine Kohlendioxidkonzentration von mindestens etwa 0,01 Gew.-%, vorzugsweise mindestens etwa 0,1 Gew.-%, angezeigt. Im Falle von medizinischen Aerosolen und insbesondere bei Inhalationsaerosolen ist im allgemeinen ein Kohlendioxidgehalt von etwa 0,01 bis 2 Gew.-%, typischerweise etwa 0,1 bis 1,0 Gew.-%, bevorzugt; höhere Konzentrationen sind in der Regel nur dann angezeigt, wenn die Formulierung einen vergleichsweise hohen Anteil an Cosolventien wie Ethanol oder Wasser enthält.

Als pharmazeutische Wirkstoffe für die erfindungsgemässen Aerosolformulierungen eignen sich grundsätzlich alle als Aerosol verabreichbaren Wirkstoffe, wie Betamimetika, Corticosteroide, Anticholinergika, Cyclooxigenase-, Mastzell-, Lipoxigenase- und Proteolytische Enzym-Inhibitoren, Arachidonsäure-, Leukotrien-, Thromboxan-, Natrium/Kaliumkanal-, Neurokinin-, Tachykinin-, Bradykinin-, Muscarin,-Histamin-, Phosphodiesterase- und Selectin-Antagonisten, Kaliumkanalblocker, Antiinfektiva, Antibiotika, Pentamidin, Cytostatika, Fungistatika, Radikalfänger, Vitamine, Hormone, Immunstimulantien, Immunsuppresiva, Mucolytika, Heparin, Antidiabetika, Analgetika, Schlafmittel und dergleichen, beispielsweise
- Betamimetika wie Salbutamol, Formoterol, Salmeterol, Salbutamol, Fenoterol, Clenbuterol, Terbutalin, Bambuterol, Broxaterol, Epinephrin, Isoprenalin, Orciprenalin, Hexoprenalin, Tulobuterol, Reproterol, Bamethan etc.,
- Corticoide wie Beclomethason, Betamethason, Ciclomethason, Dexamethason, Triamcinolon, Budesonid, Butixocort, Ciclesonid, Fluticason, Flunisolid, Icomethason, Mometason, Tixocortol, Loteprednol etc.,
- Anticholinergika und Spasmolytika wie Atropin, Scopolamin, N-Butylscopolamin, Trospiumchlorid, Ipratropiumbromid, Oxitropiumbromid, Thiotropiumbromid, Droferin, Oxybutinin, Moxaverin, Glycopyrrolat etc.,
- Mastzellinhibitoren wie Cromoglycinsäure, Nedocromil etc. und Lipoxigenasehemmer wie Zileuton,
- Leukotrienantagonisten wie Iralukast, Zafirlukast und Pranlukast, Natriumkanalantagonisten wie Amilorid, Kaliumkanalantagonisten wie Bimakalim, Arachidonsäureantagonisten wie 2-Benzoxazolamin, Histaminrezeptorantagonisten wie Epinastin, Cetrizin, Mizolastin und Mequitamium,
- Migränemittel wie Mutterkornalkaloide, Methysergid, Ergotamin, Serotonin, Sumatriptan, Zolmitriptan, Cyclandelat etc.,
- Analgetika wie Fentanyl, Morphin, Buprenorphin, Opium, Heroin, Nalbuphin, Pentazocin, Oxycodon, Tramadol, Pethidin, Tilidin, Methadon, Nefopam, Dextropropoxyphen, Piritramid etc.,
- Mucolytica wie R-nase, Acetylcystein, Ambroxol, Apafant, Bromhexin, Surfactant etc.,
- Antiemetika wie Bromoprid, Domperidon, Metoclopramid, Triethylperazin, Trifluorpromazin, Meclozin, Chlorphenoxamin, Dimenhydrinat etc.,
- Antibiotika wie Penicilline (z.B. Aziocillin), Cephalosporine (z.B. Cefotiam oder Ceftriaxon), Carbapeneme, Monobatame, Aminoglykoside (z.B. Streptomycin, Neomycin, Gentamycin, Amikacin oder Tobramycin), Chinolone (z.B. Ciprofloxacin), Makrolide (z.B. Erytromycin), Nitroimidazole (z.B. Tinidazol), Lincosamide (z.B. Clindamycin), Glykopeptide (z.B. Vancomycin), Polypeptide (z.B. Bacitracin) etc.,
- Vitamine und Radikalfänger wie Vitamin A, B, C, D oder E, Katalase, Superoxidbismutase, reduziertes Glutathion etc.,
- Antidiabetika wie Gibenclamid, Glipizid, Gliclacid, Glimepirid, Troglitazone etc.,
- Schlafmittel wie Benzodiazepine, Piperidindione, Antihistaminika etc.,
- Neuroleptika, Antidepressiva und Antikonvulsiva wie Benzodiazepine, Phenothiazine, Butyrophenone, Sulpirid, Hydantoine, Barbiturate, Succinimide, Carbamazepin etc.,
- Hormone wie Androgene (z.B. Testosteron), Antioestrogene, Oestrogene (z.B. Estradiol), Gestagene (z.B. Progesteron), Corticosteroide, Calcitonin, Parathyrin, Somatotoropin, Oxytocin, Prolactin, Glucagon, Erythropoietin, Atriopeptin, Melanotropin, Throtropin, Gonadotropin, Vasopressin, Insulin etc.,
- Potenzmittel wie Alprostadil,
- Cytostatika wie Stickstofflostderivate (z.B. Ifosphamid), N-Nitrosoharnstoffderivate (z.B. Lomustin), Antagonisten von Purin-und Pyrimidinbasen (z.B. Fluorouracil), Platinkomplexe (z.B. Carboplatin), Anthracycline (z.B. Doxorubicin), Podophyllinderivate (Podophyllotoxin)

Die genannten Wirkstoffe können gegebenenfalls in Form ihrer Isomere, Enantiomere oder Racemate und im Falle von Säuren oder Basen als solche oder in Form ihrer pharmazeutisch annehmbaren Salze verwendet werden. Die optimale Wirkstoffmenge in den erfindungsgemässen Formulierungen hängt vom jeweiligen Wirkstoff ab. In der Regel sind jedoch Aerosolformulierungen bevorzugt, die mindestens etwa 0,0001 und höchstens etwa 5 Gew.-%, insbesondere etwa 0,01 bis 3 Gew.-%, an Wirkstoff enthalten.

Die Herstellung der erfindungsgemässen Aerosolformulierungen kann in an sich bekannter Weise dadurch erfolgen, dass man Kohlendioxid unter Druck in ein verflüssigtes Hydrofluoralkan der Formel I einleitet und den pharmazeutischen Wirkstoff zusetzt. Die Zugabe des Kohlendioxids und des Wirkstoffes kann grundsätzlich in beliebiger Reihenfolge erfolgen. Im Falle von Suspensionsformulierungen ist es jedoch in der Regel bevorzugt, zuerst das Kohlendioxid in das Treibmittel einzuleiten und dann den mikronisierten Wirkstoff zuzusetzen. Die Mikronisierung des Wirkstoffes kann in bekannter Weise erfolgen und wird vorzugsweise so durchgeführt, dass eine Partikelgrösse von etwa 0,5 bis 6 µm erhalten wird.

Die Treibmittelgemische und Aerosolformulierungen können ein oder mehrere Hydrofluoralkane und gewünschtenfalls weitere Treibgase enthalten. Vorzugsweise enthalten sie jedoch keine Fluorchlorkohlenwasserstoffe. Besonders bevorzugt sind im allgemeinen solche Treibmittelgemische und Aerosolformulierungen, die - abgesehen von gewünschtenfalls als Cosolventien verwendbaren Verbindungen wie Wasser, niedere Alkane, niedere Alkohole und niedere Ether - als Treibgase lediglich Kohlendioxid und ein oder mehrere Hydrofluoralkane der Formel I enthalten. Das Hydrofluoralkan bzw. die Hydrofluoralkane und die Kohlendioxidkonzentration werden vorzugsweise so gewählt, dass im Aerosolbehältnis ein Binnendruck von etwa 3 bis 12 bar, besonders bevorzugt etwa 4 bis 7 bar, bei 20°C eingestellt werden kann.

Die erfindungsgemässen Aerosolfomulierungen. eignen sich für Suspensions- und Lösungsformulierungen, und sie können übliche Zusätze wie Cosolventien und oberflächenaktive Mittel enthalten. Die Zugabe des Wirkstoffes und allfälliger weiterer Zusätze kann in an sich bekannter Weise erfolgen. Infolge der erfindungsgemäss erzielbaren Verbesserung der Fine Particle Fraction und der damit erreichbaren hohen respirablen Fraktionen von bis zu 70% ist es häufig möglich, die Wirkstoffkonzentration um bis zu etwa 50% zu verringern.

Die Verwendung eines Cosolvens ist insbesondere bei Lösungsformulierungen häufig angezeigt, kann aber gelegentlich auch bei Suspensionsformulierungen von Vorteil sein. Als Cosolventien eignen sich insbesondere Wasser, niedere Alkohole, niedere Alkane und niedere Ether, vorzugsweise Wasser, Alkohole mit 1 bis 3 Kohlenstoffatomen, Alkane mit 3 bis 6 Kohlenstoffatomen und Dialkylether mit 2 bis 4 Kohlenstoffatomen, wie Wasser, Ethanol, Propanol, Ethylenglykol, Propylenglykol, Propan, Butan, Isobutan, Pentan, Dimethylether, Diethylether und dergleichen. Besonders bevorzugt ist Ethanol. Der Anteil an Cosolvens in den erfindungsgemässen Aerosolformulierungen kann, falls vorhanden, im allgemeinen etwa 0,1 bis 50 Gew.-%, insbesondere etwa 1 bis 30 Gew.-%, bezogen auf das Gesamtgemisch bzw. die Gesamtformulierung betragen. In den meisten Fällen genügt (falls Cosolvens nötig oder erwünscht ist) bereits eine Cosolvenskonzentration von etwa 0,1 bis 30 Gew.-%, typischerweise etwa 0,1 bis 10 Gew.-%.

Der Anteil an einem oder mehreren Hydrofluoralkanen der Formel I in den Treibmittelgemischen und Aerosolformulierungen kann dementsprechend im allgemeinen mindestens etwa 40 Gew.-%, vorzugsweise mindestens etwa 64 Gew.-% und besonders bevorzugt mindestens etwa 87 Gew.-% des Gesamtgemisches bzw. der Gesamtformulierung betragen. Im Falle der medizinischen Aerosolformulierungen kann jedoch der Anteil an Hydrofluoralkanen im Hinblick auf den Gehalt an Wirkstoff, oberflächenaktivem Mittel und allfälligen weiteren Zusätzen auch niedriger sein und beispielsweise mindestens etwa 29 Gew.-% betragen.

Die Verwendung eines oberflächenaktiven Mittels ist insbesondere bei Suspensionsformulierungen häufig angezeigt, kann aber auch bei Lösungsformulierungen z.B. zur Ventilschmierung von Vorteil sein. Grundsätzlich eignen sich alle gebräuchlichen oberflächenaktiven Mittel wie Ölsäure, Sorbitantrioleat, Cetylpyridiniumchlorid, Soyalecithin, Polyoxyethylen(20)sorbitanmonolaurat, Polyoxethylen(10)stearylether, Polyoxyethylen(2)oleylether, Polyoxyethylen(20)sorbitanmonostearat, Polyoxyethylen(20)sorbitanmonooleat, Polyoxypropylen-polyoxyethylen-Blockcopolymere, Polyoxypropylen-polyoxyethylen-ethylendiamin-Blockcopolymere, ethoxyliertes Ricinusöl und dergleichen. Bevorzugt sind im allgemeinen Ölsäure, Sorbitantrioleat und Lecithin. Der Anteil an oberflächenaktivem Mittel kann, falls vorhanden, im allgemeinen etwa 0,001 bis 5 Gew.-%, vorzugsweise etwa 0,01 bis 1 Gew.-%, bezogen auf die Gesamtformulierung, betragen. Dank den verbesserten Benetzungseigenschaften des Treibmittelgemisches sind jedoch oft bereits Konzentrationen von etwa 0,1 Gew.-% oder weniger ausreichend.

Gewünschtenfalls kann die erfindungsgemässe Aerosolformulierung ferner Wasser, beispielsweise in-einer Menge von 0,0001 bis 20 Gew.-%, bezogen auf die Gesamtformulierung, enthalten, was bei gewissen Wirkstoffen von Vorteil sein kann.

Weiterhin können die erfindungsgemässen Aerosolformulierungen gewünschtenfalls Puffersubstanzen oder Stabilisatoren wie Citronensäure, EDTA, Vitamin E und dergleichen enthalten. Im allgemeinen werden solche Substanzen, falls vorhanden, in Mengen von nicht mehr als etwa 1 Gew.-%, beispielsweise etwa 0,0001 bis 1 Gew.-%, bezogen auf die Gesamtformulierung, verwendet.

Mit dem erfindungsgemäss verwendbaren Treibgas-System lässt sich beispielsweise ein Beclomethason-Dosieraerosol herstellen, das im Vergleich zu einem FCKW-haltigen Handelsprodukt (Becotide®, Glaxo Pharmaceuticals, Grossbritannien) eine weitaus bessere Dosiergenauigkeit und eine ca. 2,7-mal so hohe FPF besitzt. Ergänzend dazu wird die Deposition im Mundrohr etwa halbiert und diejenige im "sample induction port" (artifizieller Oropharynx) von ca. 50% auf 20% reduziert. Die erfindungsgemässe Beclomethason-Formulierung ermöglicht also das Dosieraerosol in Bezug auf mehrere Aspekte vorteilhafter zu gestalten, da eine Erhöhung der respirablen Dosis bei gleichzeitiger Erniedrigung des Wirkstoffverlustes in Mundrohr, und einer Reduktion der oropharyngealen Deposition, quasi eine signifikante Effizienzsteigerung gegenüber dem Handelprodukt Becotide ermöglicht.

Mit der vorliegenden Erfindung ist es auch möglich z.B. Migränemittel per inhalationem systemisch zu verabreichen. Migränepatienten, die sehr häufig unter Erbrechen leiden, können damit alternativ behandelt werden. Mit den erfindungsgemässen Formulierungen für Dosieraerosole ist es also möglich, Wirkstoffe oder Wirkstoffkombinationen als Aerosole mit definierten Eigenschaften zu schaffen. Im Vergleich zu Handelsprodukten kann man das Partikelgrössenverteilungsspektrum so einstellen, dass man quasi den Wirkstoff in Form eines "Drug Targeting" zielgerichtet an dem gewünschten Ort in der Lunge deponieren kann. Mit dieser Art einer optimierten Wirkstoffapplikation können gleichzeitig unerwünschte Begleitwirkungen reduziert werden. Darüberhinaus können auch Wirkstoffe, die bislang nicht pulmonal appliziert wurden bzw. werden und oral nicht verabreicht werden können, da sie peroral eine schlechte Bioverfügbarkeit und/oder ein unerwünschtes Nebenwirkungsspektrum besitzen, mittels Inhalation systemisch appliziert werden, was völlig neue Therapiemöglichkeiten eröffnet. Die Erfindung dieses "Pulmonalen Drug Targeting Systems" (PDTS) wird in den Ausführungsbeispielen anhand einiger Rezepturbeispiele weiter erläutert.

Die erfindungsgemässen Aerosolformulierungen können in üblichen drucksicheren Behältnissen mit handelsüblichen Dosierventilen mit Volumina von 25 µl bis 100 µl verschlossen und mit geeigneten handelsüblichen Mundrohradaptern vernebelt werden.

Die Erfindung wird durch die folgenden Beispiele weiter veranschaulicht. Die Homogenisierung von Wirkstoffsuspensionen erfolgte jeweils mit einem Rotor-Stator-Homogenisator (Kinematika).

### Beispiel 1

a) 2,5 g Beclomethason-dipropionat werden in einen Druckansatzkessel eingewogen und in 55 g Ethanol, in dem zuvor 0,25 g Ölsäure gelöst wurden, unter Rühren gelöst. Nach Verschliessen und Evakuieren desselben werden 1,5 kg HFA 227, das zuvor in einem anderen Druckansatzkessel mit CO₂ begast und auf einen Druck von 6,5 bar (20°C) eingestellt wurde, unter Rühren zugegeben. Die erhaltene Lösung wird mittels Druckfülltechnik (Anlage von Pamasol W. Maeder, Pfäffikon, Schweiz) in mit Dosier-Ventilen verschlossene Aluminiumdosen abgefüllt.
Die erhaltene Formulierung wurde mit den auf FCKW-Treibmitteln basierenden Handelsprodukten Becotide® 100 und Becotide® 200 (Glaxo Pharmaceuticals, Grossbritannien; Batch-Nr. 10072926 bzw. 10073077) sowie Becloforte® (Glaxo AG, Schönbühl, Schweiz; Batch-Nr. 5H052) verglichen, von denen mehrere Proben in einer Apotheke gekauft und bis zur Messung in horizontaler Lage bei Raumtemperatur aufbewahrt wurden. Die neuen Formulierungen wurden in unterschiedlicher Lage (Ventil nach unten oder oben) bei 25°C und 60% relativer Feuchte bis zur Messung aufbewahrt bzw. vorher während 6 Monaten bei 30°C und 70% relativer Luftfeuchtigkeit (30°/70%rh) gelagert. Zur Bestimmung der Content Uniformity wurden im Falle der Handelsprodukte jeweils die Hübe Nr. 5-9 und 196-200 (je 3 Kanister) und im Falle der neuen Formulierung Hübe Nr. 3 und 200 (je 10 Kanister) gemessen. Die aerodynamische Partikelgrössenverteilung und die Fine Particle Fraction FPF bzw. die Fine Particle Dose FPD (Stufen 2-8) wurde mit einem 8-Stufen-Andersen-Kaskaden-Impaktor gemäss USP 23 unter Verwendung der Hübe Nr. 11-30 und 178-197 (je 3 Kanister, manuelle Betätigung) bestimmt. Die Bestimmung des Beclomethason-dipropionats erfolgt in allen Fällen mittels HPLC und UV-Messung bei 230 nm. Der mittlere aerodynamische Teilchendurchmesser MMAD wurde aus den entsprechenden logarithmischen Darstellungen der Wahrscheinlichkeitsverteilung berechnet. Die erhaltenen Werte (Anzahl Messungen in Klammer) und die relativen Standardabweichungen (RSD) sind in Tabelle 1 zusammengefasst.

**Tabelle 1**

| Produkt | Becotide | | Becloforte | Beispiel 1 | |
|---|---|---|---|---|---|
| deklarierter Wert pro Hub | 100 µg | 200 µg | 250 µg | 100 µg | 100 µg |
| Lagerung | nicht definiert | nicht definiert | nicht definiert | nein* | 6 Monate, 30°/70%rh |
| Masse/Hub | 79,3 mg | 86,9 mg | 87,9 mg | 68,8 mg | 67,9 mg |
| RSD | 13,3% | 8,1% | 3,3% | 2,9% | 5,3% |
| (n) | (30) | (30) | (30) | (20) | (40) |
| Dosis/Hub | 107,8 µg | 221,5 µg | 279,5 µg | 97,7 µg | 102,9 µg |
| RSD | 23,9% | 6,3% | 12,8% | 4,1% | 13,4% |
| (n) | (30) | (30) | (30) | (20) | (40) |
| USP-throat | 55,2 µg | 122,9 µg | 160,0 µg | 18,9 µg | 13,0 µg |
| FPD (n=6) | 26,9 µg | 45,4 µg | 47,9 µg | 61,5 µg | 71,8 µg |
| MMAD | ≈ 3,2 µm | ≈ 3,2 µm | ≈ 3,2 µm | ≈ 1,3 µm | ≈ 1,3 µm |

| | | | | | |
|---|---|---|---|---|---|
| * Ausgangsuntersuchung | | | | | |

b) In gleicher Weise wie in Absatz a) wurde eine Lösungsaerosolformulierung von Beclomethason-dipropionat in HFA 227, Ethanol und Ölsäure hergestellt, aber der Druck durch Einleiten von Kohlendioxid auf 4,5 bar (20°C) eingestellt, und die erhaltene Lösung mittels Druckfülltechnik in mit Dosier-Ventilen verschlossene Aluminiumdosen abgefüllt.

### Beispiel 2

2,25 g mikronisiertes Ipratropiumbromid werden in einen Druckansatzkessel eingewogen. Nach Verschliessen und Evakuieren desselben werden 10,5 kg HFA 227 zugegeben, das zuvor in einem anderen Druckansatzkessel mit CO₂ begast und auf einen Druck von 6 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 3

2,25 g mikronisiertes Ipratropiumbromid und 11,25 g mikronisiertes Salbutamol werden in einen Druckansatzkessel eingewogen. Nach Verschliessen und Evakuieren desselben werden 10,5 kg HFA 227 zugegeben, das zuvor in einem anderen Druckansatzkessel mit CO₂ begast und auf einen Druck von 6,25 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 4

2 g Fluticason-propionat und 0,02 g δ-Tocopherol werden in einen Druckansatzkessel eingewogen. Nach Verschliessen und Evakuieren des Ansatzkessels werden 1,5 kg HFA 134a, das zuvor in einem anderen Druckansatzkessel mit CO₂ begast und auf einen Druck von 4,5 bar (20°C) eingestellt wurde, unter Rühren zugegeben. Die erhaltene Suspension wird mittels Druckfülltechnik in mit Dosier-Ventilen verschlossene Aluminiumdosen abgefüllt.

### Beispiel 5

5,0 g mikronisiertes Budesonid werden in einen Druckansatzkessel eingewogen. Nach Verschliessen und Evakuieren desselben wird eine Mischung aus 0,85 kg HFA 134a und 0,85 kg HFA 227 zugegeben, die zuvor in einem anderen Druckansatzkessel mit CO₂ begast und auf einen Druck von 6,5 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 6

4,5 g mikronisiertes Oxitropiumbromid und 0,675 g mikronisiertes Formoterol-fumarat werden in einen Druckansatzkessel eingewogen. Nach Verschliessen und Evakuieren desselben werden 10,5 kg HFA 227 zugegeben, das zuvor in einem anderen Druckansatzkessel mit CO₂ begast und auf einen Druck von 6,25 bar (20°C) eingestellt wurde. Nach Homogenisieren dieser Mischung wird die erhaltene Suspension mittels Druckfülltechnik in mit Dosier-Ventilen verschlossene Aluminiumdosen abgefüllt.

### Beispiel 7

112,5 g mikronisiertes Lomustin werden in einen Druckansatzkessel eingewogen. Nach Verschliessen und Evakuieren desselben werden 10,5 kg HFA 227 zugegeben, das zuvor in einem anderen Druckansatzkessel, in dem 312 g Ethanol vorgelegt wurden, mit CO₂ begast und auf einen Druck von 4,5 bar (20°C) eingestellt wurde. Nach Homogenisieren dieser Mischung wird die erhaltene Formulierung mittels Druckfülltechnik in mit Dosier-Ventilen verschlossene Aluminiumdosen abgefüllt.

### Beispiel 8

5 g Heparin werden in einen Druckansatzkessel eingewogen und in 50 g Ethanol, in dem zuvor 0,25 g Lecithin gelöst wurden, unter Rühren suspendiert. Nach Verschliessen und Evakuieren desselben werden 1,5 kg HFA 227, das zuvor in einem anderen Druckansatzkessel mit CO₂ begast und auf einen Druck von 4,5 bar (20°C) eingestellt wurde, unter Rühren zugegeben und homogenisiert. Die erhaltene Suspension wird mittels Druckfülltechnik in mit Dosier-Ventilen verschlossene Aluminiumdosen abgefüllt.

### Beispiel 9

2,6 g Estradiol werden in einen Druckansatzkessel eingewogen und in 405,6 g Ethanol, in dem zuvor 0,26 g Ölsäure gelöst wurden, unter Rühren gelöst. Nach Verschliessen und Evakuieren desselben werden 6,7 kg HFA 134a, das zuvor in einem anderen Druckansatzkessel mit CO₂ begast und auf einen Druck von maximal 6,5 bar (20°C) eingestellt wurde, unter Rühren zugegeben. Die erhaltene Formulierung wird mittels Druckfülltechnik in mit Dosier-Ventilen verschlossene Aluminiumdosen abgefüllt.

### Beispiel 10

2,6 g Fentanyl werden in einen Druckansatzkessel eingewogen und in 405,6 g Ethanol, in dem zuvor 0,26 g Ölsäure gelöst wurden, unter Rühren gelöst. Nach Verschliessen und Evakuieren desselben werden 6,7 kg HFA 134a, das zuvor in einem anderen Druckansatzkessel mit CO₂ begast und auf einen Druck von maximal 6,5 bar (20°C) eingestellt wurde, unter Rühren zugegeben. Die erhaltene Formulierung wird mittels Druckfülltechnik in mit Dosier-Ventilen verschlossene Aluminiumdosen abgefüllt.

### Beispiel 11

2,6 g Scopolamin werden in einen Druckansatzkessel eingewogen und in 405,6 g Ethanol, in dem zuvor 0,26 g Ölsäure gelöst wurden, unter Rühren gelöst. Nach Verschliessen und Evakuieren desselben werden 6,7 kg HFA 134a, das zuvor in einem anderen Druckansatzkessel mit CO₂ begast und auf einen Druck von 8 bar (20°C) eingestellt wurde, unter Rühren zugegeben. Die erhaltene Lösung wird mittels Druckfülltechnik in mit Dosier-Ventilen verschlossene Aluminiumdosen abgefüllt.

### Beispiel 12

2,6 g Sumatriptan werden in einen Druckansatzkessel eingewogen und in 405,6 g Ethanol, in dem zuvor 0,26 g Ölsäure gelöst wurden, unter Rühren gelöst. Nach Verschliessen und Evakuieren desselben werden 6,7 kg HFA 134a, das zuvor in einem anderen Druckansatzkessel mit CO₂ begast und auf einen Druck von 7 bar (20°C) eingestellt wurde, unter Rühren zugegeben. Die erhaltene Zubereitung wird mittels Druckfülltechnik in mit Dosier-Ventilen verschlossene Aluminiumdosen abgefüllt.

### Beispiel 13

15,6 g Beclomethasondipropionat werden in 811 g Ethanol, das 3 g Ölsäure enthält, gelöst. Die klare Lösung wird mit 7,3 kg HFA 227 versetzt. Die erhaltene Mischung wird zu 9,4 g vorgelegtem Salbutamolsulfat gegeben und ausreichend homogenisiert. Nach Abschluss der Homogenisierung wird mit 2 kg HFA 227, das mit CO₂ begast und auf einen Druck von 5 bar (20°C) eingestellt wurde, verdünnt und abschliessend homogenisiert. Die fertige Zubereitung wird mittels Druckfülltechnik in mit Dosier-Ventilen verschlossene Aluminiumdosen abgefüllt.

### Beispiel 14

20 g Triamcinolonacetonid werden in 1,5 kg Ethanol gelöst. Die Lösung wird in offene Aluminiumdosen abgefüllt und diese mit geeigneten Dosier-Ventilen verschlossen. Die Dosen werden mit insgesamt 4 kg HFA 227, das mit CO₂ begast und auf einen Druck von 5 bar (20°C) eingestellt wurde, mittels Druckfülltechnik befüllt.

## Patentansprüche

1. Medizinische Aerosolformulierung, umfassend eine wirksame Menge eines pharmazeutischen Wirkstoffes und ein druckverflüssigtes Treibmittelgemisch, enthaltend Kohlendioxid und ein Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist.

2. Aerosolformulierung nach Anspruch 1, **dadurch gekennzeichnet**, sie mindestens 29 Gew.-%, vorzugsweise mindestens 40 Gew.-%, an Hydrofluoralkan der Formel I enthält.

3. Aerosolformulierung nach Anspruch 1 oder 2, **dadurch gekennzeichnet, dass** sie mindestens 64 Gew.-%, vorzugsweise mindestens 87 Gew.-%, an Hydrofluoralkan der Formel I enthält.

4. Aerosolformulierung nach einem der Ansprüche 1 bis 3, **dadurch gekennzeichnet, dass** sie als Hydrofluoralkan der Formel I 1,1,1,2-Tetrafluorethan, 1,1,1,2,3,3,3-Heptafluorpropan oder ein Gemisch der beiden enthält.

5. Aerosolformulierung nach einem der Ansprüche 1 bis 4, **dadurch gekennzeichnet, dass** sie bei 20°C einen Druck von 3 bis 12 bar, vorzugsweise 4 bis 7 bar, aufweist.

6. Aerosolformulierung nach einem der Ansprüche 1 bis 5, **dadurch gekennzeichnet, dass** der Kohlendioxidgehalt 0;0001 bis 10 Gew.-%, vorzugsweise 0,01 bis 6 Gew.-%, beträgt.

7. Aerosolformulierung nach einem der Ansprüche 1 bis 6, **dadurch gekennzeichnet, dass** sie zusätzlich ein Cosolvens in einer Menge von 0,1 bis 50 Gew.-%, vorzugsweise 1 bis 30 Gew.-%, enthält.

8. Aerosolformulierung nach Anspruch 7, **dadurch gekennzeichnet, dass** sie als Cosolvens Wasser, Ethanol, Propanol, Ethylenglykol, Propylenglykol, Propan, Butan, Isobutan, Pentan, Dimethylether oder Diethylether enthält.

9. Aerosolformulierung nach einem der Ansprüche 1 bis 8, **dadurch gekennzeichnet, dass** sie als Wirkstoff ein Betamimetikum, vorzugsweise Salbutamol, Formoterol, Salmeterol, Salbutamol, Fenoterol, Clenbuterol, Terbutalin, Bambuterol, Broxaterol, Epinephrin, Isoprenalin, Orciprenalin, Hexoprenalin, Tulobuterol, Reproterol oder Bamethan, enthält.

10. Aerosolformulierung nach einem der Ansprüche 1 bis 8, **dadurch gekennzeichnet, dass** sie als Wirkstoff ein Corticoid, vorzugsweise Beclomethason, Betamethason, Ciclomethason, Dexamethason, Triamcinolon, Budesonid, Butixocort, Ciclesonid, Fluticason, Flunisolid, Icomethason Mometason, Tixocortol oder Loteprednol, enthält.

11. Aerosolformulierung nach einem der Ansprüche 1 bis 8, **dadurch gekennzeichnet, dass** sie als Wirkstoff ein Anticholinergikum und/oder ein Spasmolytikum, vorzugsweise Atropin, Scopolamin, N-Butylscopolamin, Trospiumchlorid, Ipratropiumbromid, Oxitropiumbromid, Thiotropiumbromid, Droferin, Oxybutinin, Moxaverin oder Glycopyrrolat, enthält.

12. Aerosolformulierung nach einem der Ansprüche 1 bis 8, **dadurch gekennzeichnet, dass** sie als Wirkstoff einen Mastzellinhibitor, vorzugsweise Cromoglycinsäure oder Nedocromil, und/oder einen Lipoxigenasehemmer, vorzugsweise Zileuton, enthält.

13. Aerosolformulierung nach einem der Ansprüche 1 bis 8, **dadurch gekennzeichnet, dass** sie als Wirkstoff ein Migränemittel, vorzugsweise ein Mutterkornalkaloid, Methysergid, Ergotamin, Serotonin, Sumatriptan, Zolmitriptan oder Cyclandelat, enthält.

14. Aerosolformulierung nach einem der Ansprüche 1 bis 8, **dadurch gekennzeichnet, dass** sie als Wirkstoff ein Analgetikum, vorzugsweise Fentanyl, Morphin, Buprenorphin, Opium, Heroin, Nalbuphin, Pentazocin, Oxycodon, Tramadol, Pethidin, Tilidin, Methadon, Nefopam, Dextropropoxyphen oder Piritramid, enthält.

15. Aerosolformulierung nach einem der Ansprüche 1 bis 8, **dadurch gekennzeichnet, dass** sie als Wirkstoff ein Hormon, vorzugsweise ein Androgen, ein Antioestrogen, ein Oestrogen, ein Gestagen, ein Corticosteroid, Calcitonin, Parathyrin, Somatotoropin, Oxytocin, Prolactin, Glucagon, Erythropoietin, Atriopeptin, Melanotropin, Throtropin, Gonadotropin, Vasopressin oder Insulin, enthält.

16. Aerosolformulierung nach einem der Ansprüche 1 bis 15, **dadurch gekennzeichnet, dass** sie ein oberflächenaktives Mittel, vorzugsweise Ölsäure, Sorbitantrioleat, Cetylpyridiniumchlorid, Soyalecithin, Polyoxyethylen(20)sorbitanmonolaurat, Polyoxethylen (10)stearylether, Polyoxyethylen(2)-oleylether, Polyoxyethylen(20)sorbitanmonostearat, Polyoxyethylen(20)sorbitanmonooleat, ein Polyoxypropylen-polyoxyethylen-Blockcopolymer, ein Polyoxypropylen-polyoxyethylenethylendiamin-Blockcopolymer oder ethoxyliertes Ricinusöl, enthält.

17. Aerosolformulierung nach Anspruch 16, **dadurch gekennzeichnet, dass** sie höchstens 5 Gew.-%, vorzugsweise höchstens 1 Gew.-%, an oberflächenaktiven Mitteln enthält.

18. Aerosolformulierung nach einem der Ansprüche 1 bis 17, **dadurch gekennzeichnet, dass** sie höchstens 0,1 Gew.-% an oberflächenaktiven Mitteln oder kein oberflächenaktives Mittel enthält.

19. Aerosolformulierung nach einem der Ansprüche 1 bis 18, **dadurch gekennzeichnet, dass** sie zusätzlich eine Puffersubstanz und/oder einen Stabilisator, vorzugsweise Citronensäure, EDTA oder Vitamin E, enthält.

20. Verfahren zur Herstellung der medizinischen Aerosolformulierung gemäss Ansprüchen 1 bis 19, **dadurch gekennzeichnet, dass** man Kohlendioxid unter Druck in ein verflüssigtes Hydrofluoralkan der allgemeinen Formel
CₓH_{y}F_{z} (I)
worin x für die Zahl 1, 2 oder 3 steht, y und z je
eine ganze Zahl ≥ 1 bedeuten und y + z = 2x + 2 ist, einleitet und den pharmazeutischen Wirkstoff zusetzt.

## Claims

1. Medicinal aerosol formulation, comprising an effective amount of a pharmaceutically active compound and a pressure-liquefied propellant mixture, containing carbon dioxide and a hydrofluoroalkane of the general formula
CₓH_{y}F_{z} (I)
in which x is the integer 1, 2 or 3, y and z
are each an integer ≥ 1 and y + z = 2x + 2.

2. Aerosol formulation according to Claim 1, **characterized in that** it contains at least 29 % by weight, preferably at least 40 % by weight, of hydrofluoroalkane of formula I.

3. Aerosol formulation according to Claim 1 or 2, **characterized in that** it contains at least 64 % by weight, preferably at least 87 % by weight, of hydrofluoroalkane of formula I.

4. Aerosol formulation according to any one of Claims 1 to 3, **characterized in that** the hydrofluoroalkane of formula I contained is 1,1,1,2-tetrafluoroethane, 1,1,1,2,3,3,3-heptafluoropropane or a mixture of the two.

5. Aerosol formulation according to any one of Claims 1 to 4, **characterized in that** at 20°C it has a pressure from 3 to 12 bar, preferably 4 to 7 bar.

6. Aerosol formulation according to any one of Claims 1 to 5, **characterized in that** the carbon dioxide content is 0.0001 to 10 % by weight, preferably 0.01 to 6 % by weight.

7. Aerosol formulation according to any one of Claims 1 to 6, **characterized in that** it additionally contains a cosolvent in an amount from 0.1 to 50 % by weight, preferably 1 to 30 % by weight.

8. Aerosol formulation according to Claim 7, **characterized in that** the cosolvent contained is water, ethanol, propanol, ethylene glycol, propylene glycol, propane, butane, isobutane, pentane, dimethyl ether or diethyl ether.

9. Aerosol formulation according to any one of Claims 1 to 8, **characterized in that** the active compound contained is a beta-mimetic, preferably salbutamol, formoterol, salmeterol, salbutamol, fenoterol, clenbuterol, terbutaline, bambuterol, broxaterol, epinephrine, isoprenaline, orciprenaline, hexoprenaline, tulobuterol, reproterol or bamethan.

10. Aerosol formulation according to any one of Claims 1 to 8, **characterized in that** the active compound contained is a corticoid, preferably beclomethasone, betamethasone, ciclomethasone, dexamethasone, triamcinolone, budesonide, butixocort, ciclesonide, fluticasone, flunisolide, icomethasone, mometasone, tixocortol or loteprednol.

11. Aerosol formulation according to any one of Claims 1 to 8, **characterized in that** the active compound contained is an anticholinergic and/or a spasmolytic, preferably atropine, scopolamine, N-butylscopolamine, trospium chloride, ipratropium bromide, oxitropium bromide, thiotropium bromide, drofenine, oxybutinine, moxaverine or glycopyrrolate.

12. Aerosol formulation according to any one of Claims 1 to 8, **characterized in that** the active compound contained is a mast cell inhibitor, preferably cromoglycic acid or nedocromil, and/or a lipoxygenase inhibitor, preferably zileuton.

13. Aerosol formulation according to any one of Claims 1 to 8, **characterized in that** the active compound contained is an antimigraine agent, preferably an ergot alkaloid, methysergide, ergotamine, serotonin, sumatriptan, zolmitriptan or cyclandelate.

14. Aerosol formulation according to any one of Claims 1 to 8, **characterized in that** the active compound contained is an analgesic, preferably fentanyl, morphine, buprenorphine, opium, heroin, nalbuphine, pentazocine, oxycodone, tramadol, pethidine, tilidine, methadone, nefopam, dextropropoxyphene or piritramide.

15. Aerosol formulation according to any one of Claims 1 to 8, **characterized in that** the active compound contained is a hormone, preferably an androgen, an antioestrogen, an oestrogen, a gestagen, a cortico-steroid, calcitonin, parathyrin, somatotropin, oxytocin, prolactin, glucagon, erythropoietin, atriopeptin, melanotropin, thyrotropin, gonadotropin, vasopressin or insulin.

16. Aerosol formulation according to any one of Claims 1 to 15, **characterized in that** it contains a surface-active agent, preferably oleic acid, sorbitan trioleate, cetylpyridinium chloride, soya lecithin, polyoxyethylene(20) sorbitan monolaurate, polyoxyethylene(10) stearyl ether, polyoxyethylene(2) oleyl ether, polyoxyethylene(20) sorbitan monostearate, polyoxyethylene(20) sorbitan monooleate, a polyoxypropylene-polyoxyethylene block copolymer, a polyoxypropylene-polyoxyethylene-ethylenediamine block copolymer or ethoxylated castor oil.

17. Aerosol formulation according to Claim 16, **characterized in that** it contains at most 5 % by weight, preferably at most 1 % by weight, of surface-active agents.

18. Aerosol formulation according to any one of Claims 1 to 17, **characterized in that** it contains at most 0.1 % by weight of surface-active agents or no surface-active agent.

19. Aerosol formulation according to any one of Claims 1 to 18, **characterized in that** it additionally contains a buffer substance and/or a stabilizer, preferably citric acid, EDTA or vitamin E.

20. Process for the production of the medicinal aerosol formulation according to Claims 1 to 19, **characterized in that** carbon dioxide is passed under pressure into a liquefied hydrofluoroalkane of the general formula
CₓH_{y}F_{z} (I)
in which x is the integer 1, 2 or 3, y and z
are each an integer ≥ 1 and y + z = 2x + 2. and the pharmaceutically active compound is added.

## Revendications

1. Formulation d'aérosol médicinale, comprenant une quantité efficace d'une substance active pharmaceutique et un mélange propulseur liquéfié sous pression, contenant du dioxyde de carbone et un hydrofluoralcane de formule générale
CₓH_{y}F_{z} (I)
dans laquelle x représente le nombre 1, 2 ou 3, y et z représent chacun un nombre entier ≥ 1 et y + z = 2x + 2.

2. Formulation d'aérosol selon la revendication 1, **caractérisée en ce qu'**elle contient au moins 29 % en poids, de préférence au moins 40 % en poids d'un hydrofluoralcane de formule I.

3. Formulation d'aérosol selon la revendication 1 ou 2, **caractérisée en ce qu'**elle contient au moins 64 % en poids, de préférence au moins 87 % en poids d'un hydrofluoralcane de formule I.

4. Formulation d'aérosol selon l'une quelconque des revendications 1 à 3, **caractérisée en ce qu'**elle contient comme hydrofluoralcane de formule I du 1,1,1,2-tétrafluoréthane, du 1,1,1,2,3,3,3-heptafluoropropane ou un mélange des deux.

5. Formulation d'aérosol selon l'une quelconque des revendications 1 à 4, **caractérisée en ce qu'**elle présente, à 20°C, une pression de 3 à 12 bar, de préférence de 4 à 7 bar.

6. Formulation d'aérosol selon l'une quelconque des revendications 1 à 5, **caractérisée en ce que** la teneur en dioxyde de carbone est de 0,0001 à 10 % en poids, de préférence de 0,01 à 6 % en poids.

7. Formulation d'aérosol selon l'une quelconque des revendications 1 à 6, **caractérisée en ce qu'**elle contient en outre un cosolvant en une quantité de 0,1 à 50 % en poids, de préférence de 1 à 30 % en poids.

8. Formulation d'aérosol selon la revendication 7, **caractérisée en ce qu'**elle contient comme cosolvant de l'eau, de l'éthanol, du propanol, de l'éthylèneglycol, du propylèneglycol, du propane, du butane, de l'isobutane, du pentane, de l'éther diméthylique ou de l'éther diéthylique.

9. Formulation d'aérosol selon l'une quelconque des revendications 1 à 8, **caractérisée en ce qu'**elle contient comme substance active un bètamimétique, de préférence du salbutamol, du formotérol, du salmétérol, du salbutamol, du fénotérol, du clenbutérol, de la terbutaline, du bambutérol, du broxatérol, de l'épinéphrine, de l'isoprénaline, de l'orciprénaline, de l'hexoprénaline, du tulobutérol, du réprotérol ou du baméthan.

10. Formulation d'aérosol selon l'une quelconque des revendications 1 à 8, **caractérisée en ce qu'**elle contient comme substance active un corticostéroïde, de préférence de la béclométasone, de la bétaméthasone, de la ciclométhasone, de la dexaméthasone, de la triamcinolone, du budesonide, du butixocort, du ciclesonide, de la fluticasone, du flunisolide, de l'icométhasone, de la mométasone, du tixocortol ou du lotéprédnol.

11. Formulation d'aérosol selon l'une quelconque des revendications 1 à 8, **caractérisée en ce qu'**elle contient comme substance active un anticholinérgique et/ou un spasmolytique, de préférence de l'atropine, de la scopolamine, de la n-butylscopolamine, du chlorure de trospium, du bromure d'ipratropium, du bromure d'oxitropium, du bromure de thiotropium, de la drofémine, de l'oxybutynine, de la moxavérine ou du glycopyrrolate.

12. Formulation d'aérosol selon l'une quelconque des revendications 1 à 8, **caractérisée en ce qu'**elle contient comme substance active un inhibiteur des mastocytes, de préférence de l'acide cromoglicique ou du nédocromil, et/ou un inhibiteur de la lipoxygénase, de préférence du zileuton.

13. Formulation d'aérosol selon l'une quelconque des revendications 1 à 8, **caractérisée en ce qu'**elle contient comme substance active un agent antimigraineux, de préférence un alcaloïde de l'ergot de seigle , du méthysergide, de l'ergotamine, de la sérotonine, du sumatriptane, du zolmitriptane ou du cyclandélate.

14. Formulation d'aérosol selon l'une quelconque des revendications 1 à 8, **caractérisée en ce qu'**elle contient comme substance active un analgésique, de préférence du fentanyl, de la morphine, de la buprénorphine, de l'opium, de l'héroïne, de la nalbuphine, de la pentazocine, de l'oxycodone, du tramadol, de la péthidine, de la tilidine, de la méthadone, du néfopam, du dextropropoxyphène ou du piritramide.

15. Formulation d'aérosol selon l'une quelconque des revendications 1 à 8, **caractérisée en ce qu'**elle contient comme substance active une hormone, de préférence un androgène, un antioestrogène, un oestrogène, un gestagène, un corticostéroïde, de la calcitonine, de la parathyrine, de la somatotropine, de l'ocytocine, de la prolactine, du glucagon, de l'érythropoïétine, de l'atriopeptine, de la mélanotropine, de la thyrotropine, de la gonadotropine, de la vasopressine ou de l'insuline.

16. Formulation d'aérosol selon l'une quelconque des revendications 1 à 15, **caractérisée en ce qu'**elle contient un agent tensioactif, de préférence de l'acide oléique, du trioléate de sorbitane, du chlorure de cétylpyridinium, de la lécithine de soja, du monolaurate de polyoxyéthylène(20)sorbitane, de l'éther stéarylique de polyoxyéthylène(10), de l'éther oléique de polyoxyéthylène(2), du monostéarate de polyoxyéthylène(20)sorbitane, du monooléate de polyoxyéthylène(20)sorbitane, un copolymère en blocs de polyoxypropylène et de polyoxyéthylène, un copolymère en blocs de polyoxypropylène et de polyoxyéthylène éthylènediamine ou de l'huile de ricin éthoxylée.

17. Formulation d'aérosol selon la revendication 16, **caractérisée en ce qu'**elle contient au plus 5 % en poids, de préférence au plus 1 % en poids d'agent tensioactif.

18. Formulation d'aérosol selon l'une quelconque des revendications 1 à 17, **caractérisée en ce qu'**elle contient au plus 0,1 % en poids d'agent tensioactif ou qu'elle ne contient pas d'agent tensioactif.

19. Formulation d'aérosol selon l'une quelconque des revendications 1 à 18, **caractérisée en ce qu'**elle contient en outre une substance tampon et/ou un agent de stabilisation, de préférence de l'acide citrique, de l'EDTA ou de la vitamine E.

20. Procédé de préparation de la formulation d'aérosol médicinal selon l'une quelconque des revendications 1 à 19, **caractérisée en ce que** l'on introduit du dioxyde de carbone sous pression dans un hydrofluoralcane liquéfié de formule générale
CₓH_{y}F_{z} (I)
dans laquelle x représente le nombre 1, 2 ou 3, y et z représentent chacun un nombre entier ≥ 1 et y + z = 2x + 2, et l'on ajoute la substance active pharmaceutique.
